# EUROPEAN PATENT APPLICATION

(11) **EP 2 106 804 A2**
(43) Date of publication of application: **07.10.2009**
(21) Application number: 08168802.0
(22) Date of filing: 11.11.2008
(51) Int. Cl.: A61K 39/395, A61K 51/00, G01N 33/50

(54) **Composition and method for detection of pre-metastatic sites**

(30) Priority: 31.03.2008 KR 20080029892
(71) Applicant: Korea Atomic Energy Research Institute, Daejeon 305-353 (KR)
(72) Inventor: Choi, Sun-Ju, 305-340, DAEJEON (KR); Hong, Young-Don, 302-777, DAEJEON (KR); Lee, So-Young, 305-340, DAEJEON (KR); Yoon, Sun-Ha, 302-754, DAEJEON (KR); Kim, Hong Kwon, 137-070, SEOUL (KR)
(74) Representative: Fiussello, Francesco

(57) **Abstract**

Disclosed are a radio-immunoconjugate for diagnosis and treatment of cancer or metastasis and development of metastasis inhibitory formulations using the same. More particularly, the present invention provides a radio-immunoconjugate as material indicating a metastatic cancer that has antibody marked with any lanthanum radionuclide and/or gamma, beta or alpha ray emitting radioisotopes targeting a vascular endothelial growth factor receptor (VEGFR). Such a radio-immunoconjugate is advantageous in that it maintains structural stability of a protein and immune activity thereof and is effectively adsorbed to the surface of vascular endothelial cells, thereby being useful as a pre-metastatic site detection factor. When the radio-immunoconjugate is administered to an animal model with a cancer, the radio-immunoconjugate is accumulated in cancer tissues, and therefore, is useful for development of radioactive metastasis inhibitory formulations.

## Description

### BACKGROUND OF THE INVENTION

This application claims priority to Korean Patent Application No. 2008-29892, filed on March 31, 2008, in the Korean Intellectual Property Office, the entire contents of which are hereby incorporated by reference.

### 1. Field of the Invention

The present invention relates to a radio-immunoconjugate for diagnosis and treatment of cancer or metastasis and development of metastasis inhibitory formulations using the same. More particularly, the present invention provides a radio-immunoconjugate as material indicating a metastatic cancer that has antibody marked with any lanthanum radionuclide and/or gamma, beta or alpha ray emitting radioisotopes targeting a vascular endothelial growth factor receptor (VEGFR). And the present invention provides composition for detection of pre-metastatic sites and cancer metastasis inhibitory formulations containing the radio-immunoconjugate.

### 2. Description of the Related Art

Radioisotope marking techniques applied to physiologically active materials are generally employed to treatment of diseases including cancers. The radioisotopes fundamentally have weak transmission but emit beta rays with strong destructive force and optionally gamma rays. Major isotopic elements used for internal irradiation treatment using seal-less radioisotopes are ⁸⁹Sr, ³²P, ⁹⁰Y, ¹⁸⁸Re, ¹⁵³Sm, ¹⁶⁶Ho and so forth, and a method of using these elements includes introducing a marking compound to emit beta energy suitable to treat a cancer so that the marking compound accumulates only in the cancer, thereby treating the same. Also, since specific nuclides with a short half-life sufficient to decay the nuclides in a reduced time are used, these elements have substantially less influence on organs or other portions of a human body excluding the cancer. In addition, the nuclide is concentrated in only one site during the treatment, thus minimizing metastasis of the cancer to other internal organs. Among the above isotopic elements, ¹⁶⁶Ho is a beta ray emitting radionuclide emitting energy at 1.77MeV (48%) and 1.85MeV (51%), which is well known to treat cancers.

Diagnostic methods using radioisotopes may include positron emission tomography (PET), photon emission computed tomography (SPECT), use of a gamma camera, and so forth. PET refers to a process comprising: combining a metabolite such as glucose with a positron emitting radioisotope; administering the combined material to a human body; observing biochemical changes that occur in the body; and forming CT images. Based on a principle wherein a tissue with cancer consumes glucose much more than other tissues and, when the cancer absorbs a relatively large amount of glucose compared to other tissues, only the cancer tissues emit radioisotopes, the PET functions to detect emission signals and to generate images from the detected signals. SPECT refers to a process comprising: intravenous (IV) administering a gamma ray emitting radioactive compound to a patient; taking pictures of blood flow distribution in organs such as heart, brain, liver, bone, etc. when the radioactive compound is entirely and homogeneously distributed throughout the organs; observing changes of the distribution caused by a disease; and forming CT images based on the observed results. The gamma camera is the equipment that: measures gamma rays emitted from a radioactive compound, which was administered to a body for the purpose of diagnosis, using a detector fixed to a test subject; and records internal distribution of the compound or distribution of the compound in organs then forms images based on the recorded results.

Growth of a new vascular network connected to cancer tissues is considered as a significant condition for cancer metastasis. Such a vascular cell growth is necessary for primary cancers and/or metastatic tumors and a cancer tissue cannot grow to a size of more than 1 to 2mm³ unless nutrients and oxygen are supplied by the vascular cell growth (see, Folkman, Cancer Biol. 1992). During the vascular cell growth, a primary cancer cell flows into a blood vessel, moves to other sites and generates a metastatic tumor. That is, conventional treatment agents targeting vascular cell growth factors may be commonly used in all solid tumors.

Recently, the most well known vascular cell growth factor is a vascular endothelial growth factor (VEGF). Studies for identifying a cancer metastasis mechanism using the VEGF have recently proceeded and, as a result, reported that a bone marrow-derived cell having a vascular endothelial growth factor receptor 1 (VEGFR 1), which is the first determinant to select a pre-metastatic site and promotes metastasis, moves to a specific site where a cancer cell inducible environment is formed (see, Rosandra Kaplan, et al., Nature, 2005). In addition, it was disclosed that when antibody targeting a VEGFR 1 is administered to an experimental animal model to which lung cancer or melanoma cells were xenografted, the antibody is combined with VEGFR 1 existing in the mouse so as to inhibit metastasis thereof (see, Rosandra Kaplan, et. al., Nature, 2005).

Accordingly, it is expected that using such VEGFR 1 in immunotherapy and/or radioimmunotherapy, there will be simultaneously achieved detection and metastasis inhibition of pre-metastatic sites.

Under circumstances described above, the present inventors found that a radio-immunoconjugate prepared by stably marking a VEGFR with a radioisotope for diagnosis and medical treatment, is efficiently adsorbed to the surface of vascular endothelial cells without alteration in immune activity and/or structure of protein, and exhibits excellent accumulation in cancer tissues in the body of an animal model used in a cancer development experiment, and therefore, identified that the radio-immunoconjugate may be used for marking, diagnosis and medical treatment of cancer as well as detection and inhibition of pre-metastasis, thereby completing the present invention.

### SUMMARY OF THE INVENTION

Accordingly, the present invention has been proposed to solve problems of conventional techniques, and an object of the present invention is to provide a composition for detection of pre-metastatic sites and inhibition of metastasis, containing a radio-immunoconjugate that has antibody marked with a radioisotope targeting a vascular endothelial growth factor receptor (VEGFR).

In order to accomplish the above object of the present invention, there is provided a composition for detection of pre-metastatic sites, containing a radio-immunoconjugate that has antibody marked with a radioisotope targeting a vascular endothelial growth factor receptor (VEGFR).

The present invention also provides a method for detection of pre-metastatic sites, comprising: (1) administering the composition for detecting pre-metastatic sites described above to an individual with a cancer; and (2) detecting signals emitted from tissues of the individual by the composition in the step (1) then imaging the detected signals.

Furthermore, the present invention provides a method for diagnosis of cancer or metastasis, comprising: (1) administering a composition containing the radio-immunoconjugate described above to an individual; (2) detecting signals emitted from tissues of the individual by the composition in the step (1) then imaging the detected signals to determine an accumulation rate thereof; and (3) comparing the determined accumulation rate in the step (2) to that of a normal individual and selecting individuals with relatively high accumulation rates.

In addition, the present invention provides a kit for diagnosis of cancer or metastasis, containing the radio-immunoconjugate described above.

Further, the present invention provides a composition for inhibition of metastasis, containing the radio-immunoconjugate described above.

Furthermore, the present invention provides a method for inhibition of metastasis, comprising administration of a therapeutically effective amount of the composition for inhibition of metastasis described above to an individual with a cancer.

The composition for detection of pre-metastatic sites that contains a radio-immunoconjugate using antibody targeting a VEGRF according to the present invention, may be used for preliminary diagnosis and treatment of pre-metastatic sites as well as treatment of a primary cancer or a metastatic tumor, so that the composition may be used as a detection factor. Alternatively, the composition for inhibition of metastasis that contains a radio-immunoconjugate, may primarily block metastasis so as to eliminate the possibility of metastasis or recurrence thereof, thereby effectively inhibiting metastasis in its early stages.

### BRIEF DESCRIPTION OF THE DRAWINGS

These and other objects, features, aspects, and advantages of the present invention will be more fully described in the following detailed description of preferred embodiments, taken in conjunction with the accompanying drawings. In the drawings:
Fig. 1 illustrates cell transformation of a normal cell through an inter-cellular network when the normal cell as well as a cancer cell were co-cultured; especially, a) is a schematic view of an experiment and b) is an electron microscope picture (40X);
Fig. 2 is a schematic view illustrating carcinogenesis observed from reduced expression of tumor suppressor proteins (p53, p21) in modified cells while increasing apoptosis inhibitory proteins (cancer cell indicating factor, Bc12);
Fig. 3 is a schematic view illustrating over-expression of FLT-1 as a VEGFR after carcinogenesis of normal cells;
Fig. 4 illustrates a stable radio-immunoconjugation after antibody marked with Lu-177 targeting a VEGFR; especially, a) is a graphical representation illustrating an instant thin-layer chromatography (TLC) profile, b) is an electrophoresis image, and c) is a radiographic picture;
Fig. 5 is a graphical representation illustrating degrees of targeting vascular endothelial cells (surface adsorption rates) by antibody marked with Lu-177 targeting the VEGFR; and
Fig. 6 is a graphical representation illustrating variation in distribution of antibody marked with Lu-177 targeting the VEGFR in an animal model used in a cancer development experiment.

### DETAILED DESCRIPTION OF THE INVENTION

Hereinafter, the present invention will be described in greater detail.

In an aspect of the present invention, there is provided composition for detection of pre-metastatic sites, containing a radio-immunoconjugate that has antibody marked with a radioisotope targeting a vascular endothelial growth factor receptor (VEGFR).

Such a VEGFR may include at least one selected from KDR, flk-1 and flt-1, however, is not particularly limited thereto.

An antibody may include a humanized antibody, a chimeric antibody, a modified antibody that was conjugated with polyethylene glycol (PEG), or a fragment thereof, however, is not particularly limited thereto.

The radioisotope used herein for marking the antibody may include at least one selected from a group consisting of Sc-47, Cu-64, Cu-67, Ga-68, Br-76, Y-86, Y-90, Tc-99m, In-111, Sm-153, Dy-165, Ho-166, Er-169, Yb-169, Lu-177, Re-186 and Re-188 and, preferably, Lu-177, however, is not particularly limited thereto.

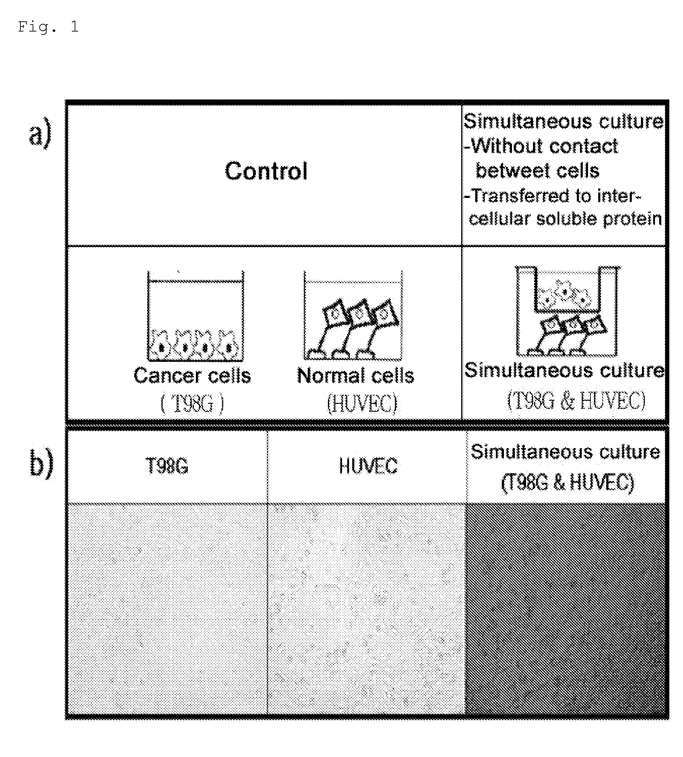

As for proving that the VEGFR can be used as a metastasis detector, the present inventors found that when a brain cancer cell and a human umbilical vein endothelial cell (HUVEC) were extracted and incubated in a same chamber, the HUVEC was transformed through an inter-cellular network (as shown in Fig. 1).

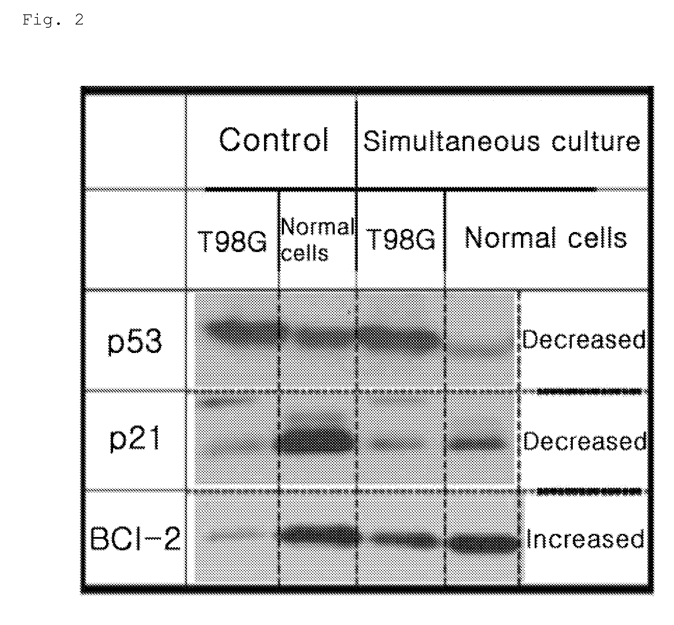

In order to discover a reason for cell transformation caused by a hybrid culture, transformed cells were recovered and used to monitor an increase in protein levels in view of p53, p21 and Bcl 2 through Western Blotting analysis. As a result, the most representative cancer inhibitory gene, P53, as well as another protein p21 that prevents cell growth when the cell is damaged, thus inhibiting cell transformation, were decreasingly expressed. On the other hand, expression of Bcl 2 protein which is well known to inhibit apoptosis and to be excessively expressed in different tumor cells, was observed to increase. From these observations, it can be seen that normal cells have been transformed into cancer cells (as shown in Fig. 2).

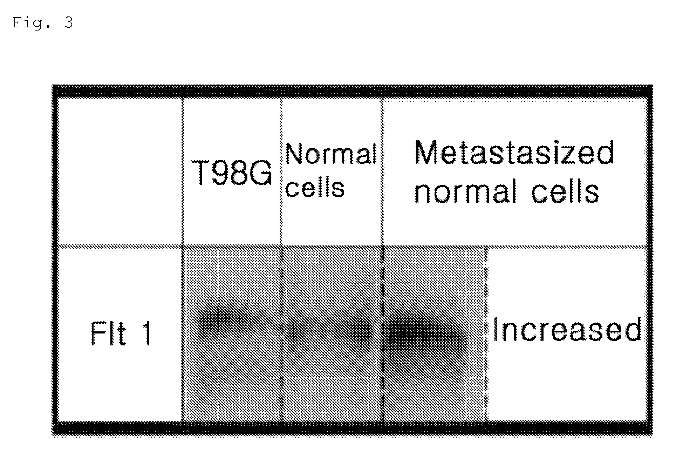

The present inventors also investigated expression degree of a specific VEGFR, Flt 1, known as the first determinant to determine pre-metastatic sites in metastasis derived cells so as verify whether the VEGFR can be utilized as a metastasis detector. As a result, it can be seen that the metastasis derived cells exhibited over-expression of Flt 1 more than that in the normal cells (as shown in Fig. 3).

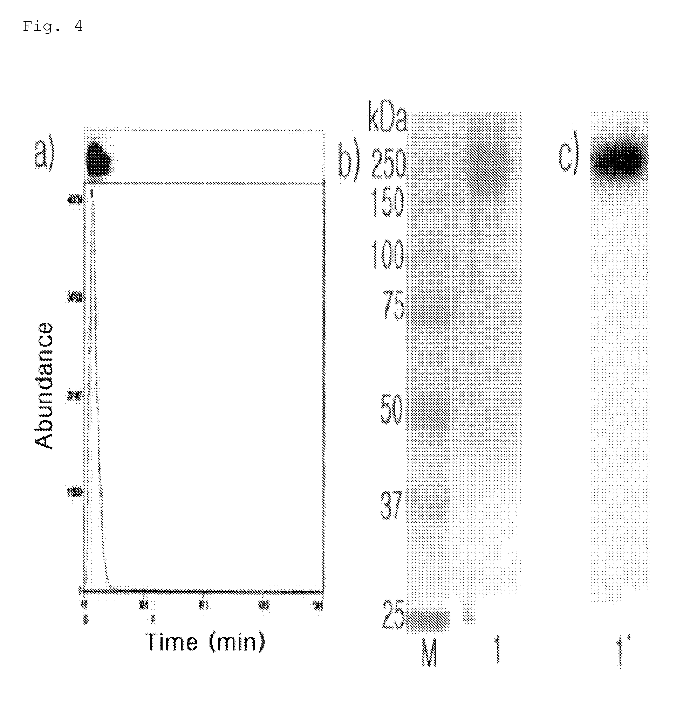

As to a condition for using the VEGFR as a metastasis detector, structural stability of a protein and immune activity were investigated. After immuno-conjugation of antibody targeting the VEGFR with a bi-functional complexing agent, the immuno-conjugated product was marked using the radioisotope Lu-177 to synthesize a radio-immunoconjugate. The radio-immunoconjugate was subjected to protein staining and a radiographic process to verify the structural stability thereof. Consequently, it can be seen that the radio-immunoconjugate maintained favorable structural stability and immune activity (as shown in Fig. 4).

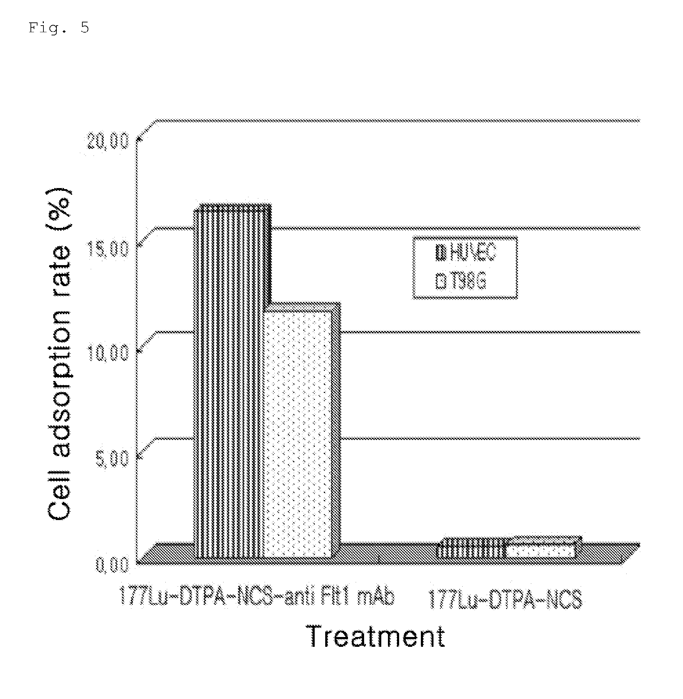

As to another condition for using the VEGFR as a metastasis detector, specific adsorption to cancer cells was investigated. After a control only having a bi-functional complexing agent marked with Lu-177 and the radio-immunoconjugate of the present invention which has the marked antibody targeting the VEGFR, respectively, were administered to each culture solution containing vascular endothelial cells, adsorption to the surface of cancer cells was determined for each case. As a result, the control only having a bi-functional complexing agent marked with Lu-177 exhibited an adsorption rate of at most 0.5%, while the inventive radio-immunoconjugate having the marked antibody targeting the VEGFR had an adsorption rate of 16.35%, thus demonstrating high target attraction to cancer cells (as shown in Fig. 5). Accordingly, it can be seen that the inventive radio-immunoconjugate exhibits excellent specific bonding to external VEG factors out of the vascular endothelial cells, thereby achieving detection of pre-metastatic sites.

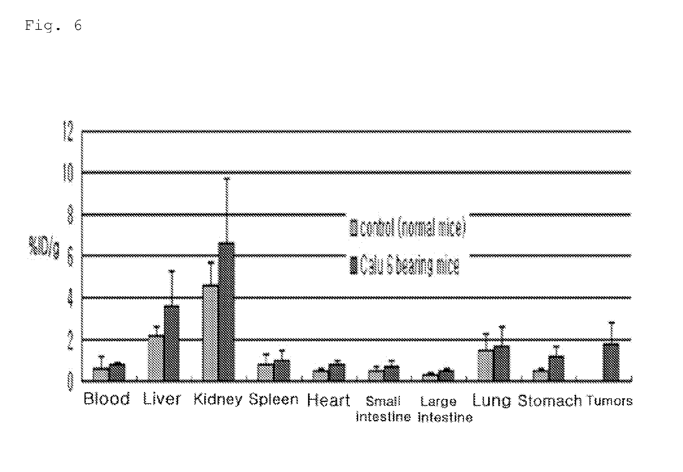

As to yet another condition for using the radio-immunoconjugate as a metastasis detector, an accumulation rate of the radio-immunoconjugate in cancer cells was investigated. After introducing the inventive radio-immunoconjugate using the antibody targeting the VEGFR into a blood vessel of an animal model used in a cancer development experiment, each organ and cancer cells were subjected to radiation measurement. As a result, it was found that the cancer cells had an accumulation rate of the radio-immunoconjugate 4.75 times that of the radio-immunoconjugate residue in blood (as shown in Fig. 6). Accordingly, it can be seen that the inventive radio-immunoconjugate may use the antibody targeting the VEGFR to target solid cancers.

The inventive radio-immunoconjugate may have an adsorption rate of 5 to 30%, preferably, 10 to 20% to the surface of the vascular endothelial cells, however, is not particularly limited thereto.

In case that the radio-immunoconjugate of the present invention is administered to an individual with a cancer, the conjugate in the cancer cells may have an accumulation rate of 2 to 10 times, preferably, 3 to 5 times that in normal tissues, however, is not particularly limited thereto.

In another aspect of the present invention, there is provided a method for detection of pre-metastatic sites, comprising: (1) administering the composition for detecting pre-metastatic sites as set forth in claim 1 to an individual with a cancer; and (2) detecting signals emitted from tissues of the individual by the composition of the step (1) then imaging the detected signals.

According to the detection method described above, the cancer in the step (1) may be selected from a group consisting of liver cancer, gastric cancer, breast cancer, colon cancer, bone cancer, pancreatic cancer, head or neck cancer, uterine cancer, ovarian cancer, rectal cancer, esophageal cancer, small intestine cancer, perianal cancer, fallopian tube cancer, endometrial cancer, cervical cancer, vaginal cancer, vulvar cancer, Hodgkin's disease, prostate cancer, bladder cancer, renal cancer, ureter cancer, renal cytoma, renal pelvis cancer and tumors of the central nervous system and the like, however, is not particularly limited thereto.

As to the detection method described above, the imaging in the step (2) may be performed by any one selected from PET, SPECT and a gamma camera, however, the imaging process is not particularly limited thereto.

In another aspect of the present invention, there is provided a method for diagnosis of cancer or metastasis, comprising: (1) administering a composition containing radio-immunoconjugate having antibody marked with a radioisotope targeting a VEGFR to an individual; (2) detecting signals emitted from tissues of the individual by the composition in the step (1) then imaging the detected signals to determine an accumulation rate thereof; and (3) comparing the determined accumulation rate in the step (2) to that of a normal individual and selecting individuals with relatively high accumulation rates.

As to the diagnosis method described above, the imaging in the step (2) may be performed by any one selected from PET, SPECT and a gamma camera, however, the imaging process is not particularly limited thereto.

As to the diagnosis method described above, the accumulation rate in the step (3) may be 2 to 10 times, preferably 3 to 5 times that in tissues of a normal individual, however the accumulation rate is not particularly limited thereto.

As to the diagnosis method describe above, the cancer in the step (3) may be selected from a group consisting of liver cancer, gastric cancer, breast cancer, colon cancer, bone cancer, pancreatic cancer, head or neck cancer, uterine cancer, ovarian cancer, rectal cancer, esophageal cancer, small intestine cancer, perianal cancer, fallopian tube cancer, endometrial cancer, cervical cancer, vaginal cancer, vulvar cancer, Hodgkin's disease, prostate cancer, bladder cancer, renal cancer, ureter cancer, renal cytoma, renal pelvis cancer and tumors of the central nervous system and the like, however, is not particularly limited thereto.

In an yet aspect of the present invention, there is provided a kit for diagnosis of cancer or metastasis, containing a radio-immunoconjugate that has antibody marked with a radioisotope targeting a VEGFR.

In addition, the present invention provides a composition for inhibiting metastasis, containing a radio-immunoconjugate that has antibody marked with a radioisotope targeting a VEGFR.

The radio-immunoconjugate of the present invention has excellent specific bonding to VEGF out of vascular endothelial cells and high accumulation rate in cancer cells so as to mark solid cancers, thereby inhibiting metastasis thereof.

The metastasis inhibitory composition of the present invention may further contain pharmaceutically available salts in addition to the radio-immunoconjugate. Such a pharmaceutically available salt may include additive salts obtained from free acid. Preferred examples of the free acid may be organic acid or inorganic acid. Such the inorganic acid may be hydrochloric acid, bromic acid, sulfuric acid, phosphoric acid and so forth. Such the organic acid may be citric acid, acetic acid, lactic acid, tartariac acid, maleic acid, fumaric acid, formic acid, propionic acid, oxalic acid, trifluoroacetic acid, benzoic acid, gluconic acid, methanesulfonic acid, glycolic acid, succinic acid, 4-toluenesulfonic acid, galacturonic acid, embonic acid, glutamic acid, aspartic acid, and so forth. Additionally, the pharmaceutically available salt may include all the salts that may be prepared by conventional methods, hydrides and solvates.

The metastasis inhibitory composition of the present invention may be orally or parenterally administered and used in the form of medical formulations. In order to prepare the formulation, any additive such as a filler, extending agent, binder, wetting agent, diluents such as surfactant and disintegrating agent, excipient may be added to the composition. A solid formulation for oral administration may include tablets, pills, powders, granulates, capsules and the like. Such a solid formulation may be prepared by adding at least one selected from, for example, starch, calcium carbonate, sucrose, lactose, gelatin, etc. to a metastasis inhibitory composition of the present invention. Other than general excipients, lubricants such as magnesium, stearate, talc and the like may also be used. A liquid formulation for oral administration may include a suspension, anti-solution agent, emulsifier, syrup and the like. In addition to typical diluents such as water, liquid paraffin, etc., a variety of excipients such as a wetting agent, sweetener, aromatic agent, preservative and the like may also be used. Meanwhile, parenteral formulations may include a sterile aqueous solution, non-aqueous solvent, suspension, emulsifier, lyophilizing agent, suppository and the like. The non-aqueous solvent and suspension may include vegetable oil such as propylene glycol, polyethylene glycol, olive oil, etc. and an injective ester such as ethyl oleate. The suppository may be prepared using a base material such as witepsol, macrogol, tween 61, cacao butter, laurin butter, glycerol, gelatin, and so forth. The cancer inhibitory composition of the present invention may be parenterally administered by subcutaneous, intravenous and/or intramuscular injection.

Administration unit may include, for example, 1, 2, 3 or 4 times each dosage, or otherwise, 1/2, 1/3 or 1/4 times each dosage. Such dosage preferably refers to an amount of an active drug per time and commonly corresponds to 1, 1/2, 1/3 or 1/4 times of a dose per day. An effective amount of the cancer inhibitory composition may range from 0.0001 to 10g/kg, preferably 0.0001 to 5g/kg and be administered 1 to 6 times per day.

Moreover, the present invention provides a method for inhibition of metastasis, comprising administration of the metastasis inhibitory composition to an individual with a cancer.

Hereinafter, exemplary embodiments of the present invention will be described in detail with reference to the following examples, which are only given for the purpose of illustration and are not to be construed as limiting the scope of the invention.

### EXAMPLE 1: Identification of inter-cellular relation between cancer cells and normal cells

Cancer cells, glioblastoma (T98G; Korean cell line bank No. 21690) were inoculated in Transwell plates (purchased from Millipore Co.) at 1×10⁶ per well located above a polycarbonate film having a 0.4 µm pore size, while the same amount of human umbilical vein endothelial cells (HUVEC) (purchased from Lonza Co.) were inoculated into wells located below the polycarbonate film. Subsequently, the inoculated samples were cultured at 37°C in 5% CO₂ atmosphere for 48 hours under constant temperature and humidity conditions, followed by observing cell modification caused by a film separation culture through an electron microscope (40X) (Leica, USA).

As a result, it was identified that the normal cells, vascular endothelial cells were transformed (see Fig. 1).

### EXAMPLE 2: Western blotting analysis

In order to examine the cell transformation caused by the film separation culturing as described in Example 1, the transformed cells were separated from the polycarbonate film after the culturing and added to 100 µℓ of a buffer solution (0.5M Tris-Cl, 0.01 MEGTA, Triton X-100; 0.4M PMSF), followed by crushing the cells at 4°C for 30 minutes. Loading the treated cells into an acrylamide gel, the mixture was subjected to electrophoresis then moved into a nitrocellulose film. Using p53, p21, Bcl 2 and Flt 1 antibodies as first antibodies (Santa Cruz Biotechnology, USA), as well as goat anti-mouse IgG-HRP (Santa Cruz Biotechnology, USA) as a second antibody, the cells were treated so as to monitor a band thereof.

As a result, it can be seen that expression of p53 and p21 proteins was reduced while expression of Bcl 2 protein was increased, and therefore, the normal cells were connected with cancer cells through inter-cellular network, leading to carcinogenesis (see Fig. 2). It was also found that expression of Flt 1 protein increased, thus demonstrating that VEGFR is applicable as a metastasis detector (see Fig. 3).

### EXAMPLE 3: Synthesis of radio-immunoconjugate marked with lanthanum radionuclide (Lu-177)

The VEGFR, Flt 1, which was identified as a pre-metastatic site determinant in Example 2 was stably marked with Lu-177 which is well known as a lanthanum radionuclide simultaneously emitting beta rays and gamma rays. The marking process was performed by dissolving an anti-Flt 1 antibody (Santa Cruz, Co. USA) targeting the VEGFR in a PBS buffer solution (pH 7.4) to prepare 0.lmM diluted solution, adding DTPA-NCS (DTPA isothiocyanate) as a bi-functional complexing agent for radio-immunoconjugation to the solution in a ratio by moles of 1:1 to produce an immunoconjugate, and purifying the obtained mixture through a ultrafiltration film (purchased from Millipore Co., Centricon filter 50kDa). Reacting the purified immunoconjugate with 0.1mCi Lu-177 solution (manufactured by HANARO, Korea Atomic Energy Research Institute) at room temperature for 5 minutes, the reaction product was treated using an instant TLC (EG&G Berthold linear analyzer) and a cyclone storage phosphor system (Perkin Elmer, USA) so as to determine a mark yield. After protein electrophoresis in a polyacrylamide gel, the resultant product was subjected to a coomassie blue staining using coomassie brilliant blue R-250 (BioRad, SIGMA, USA) and, at the same time, a radiography analysis using the cyclone storage phosphor system (Perkin Elmer, USA), so as to identify structural stability of the radio-immunoconjugate.

From the identification, neither degradation of the antibody nor generation of other side products were observed whereas Lu-177 marked bands were observed. It can be seen that the synthesized radio-immunoconjugate favorably maintained structural stability and immune activity (see Fig. 4).

### EXAMPLE 4: Determination of cell surface adsorption of radio-immunoconjugate

Antibody marked with Lu-177 targeting the VEGFR (¹⁷⁷Lu-DTPA-NCS-anti Flt 1 mAb) resulted from Example 3 was used to determine surface adsorption of the HUVEC (Lonza Co.). Administering the radio-immunoconjugate (5 uCi Lu-177, containing 1 ug of antibody) to a culture solution containing HUVEC, the mixture was left at 37°C for 1 hour under a constant temperature condition. Washing the mixture with the PBS solution (pH 7.4), cells were recovered and undertook comparison and analysis of amount of the radio-immunoconjugate to be adsorbed to the surface of the cells using a gamma counter (Perkin Elmer, USA).

As a result, if the bi-functional complexing agent was only marked with Lu-177, the radio-immunoconjugate exhibited an adsorption rate of at most 0.5%. On the other hand, when the antibody targeting the VEGFR was marked, the adsorption rate of the radio-immunoconjugate reached 16.35%. In addition, the radio-immunoconjugate exhibited excellent target attraction to human brain cancer cells. Therefore, it can be seen that the radio-immunoconjugate has excellent target attraction to any VEGFR existing in pre-metastatic sites (see Fig. 5).

### EXAMPLE 5: Observation of internal distribution of radio-immunoconjugate in cancer-induced animal model

Radio-immunoconjugate marked with Lu-177 targeting the VEGFR (¹⁷⁷Lu-DTPA-NCS-anti Flt 1 mAb) resulted from Example 3 was used to identify internal distribution of the antibody in an experimental animal. A female nude mouse weighting 21 to 23g that was xenografted with human lung cancer cells, (obtained from Orientbio Inc., KOREA) was used as the experimental animal. 5 uCi of the radio-immunoconjugate (containing 1 µg of antibody) was injected into a tail vein of the mouse. After 24 hours, organs (including blood, heart, lung, liver, spleen, stomach, small intestine, large intestine, kidney) and cancer tissues were excised and weighed, followed by measuring radioactivity in each organ using a gamma counter. The measured results were applied to calculation of injected dose per g weight of the organ, that is, percent of injected dose/g (% ID/g).

As a result, compared to the injected dose remaining in blood of 0.32% ID/g, the injected dose remaining in cancer tissues was 1.56% ID/g, thus verifying relatively high accumulation rate. Therefore, it can be seen that the synthesized radio-immunoconjugate of the present invention is useful for diagnosis and treatment of cancer or metastasis as well as diagnosis of pre-metastatic sites.

Although the present invention has been described in detail reference to its presently preferred embodiment, it will be understood by those skilled in the art that various modifications and equivalents can be made without departing from the spirit and scope of the present invention, as set forth in the appended claims.

## Claims

1. A composition for detection of pre-metastatic sites, comprising a radio-immunoconjugate that has antibody marked with a radioisotope targeting a vascular endothelial growth factor receptor (VEGFR).

2. The composition according to claim 1, wherein the VEGFR is at least one selected from KDR, flk-1 and flt-1.

3. The composition according to claim 1, wherein the antibody is selected from a humanized antibody, a chimeric antibody and a fragment thereof.

4. The composition according to claim 1, wherein the radioisotope is at least one selected from a group consisting of Sc-47, Cu-64, Cu-67, Ga-68, Br-76, Y-86, Y-90, Tc-99m, In-111, Sm-153, Dy-165, Ho-166, Er-169, Yb-169, Lu-177, Re-186 and Re-188.

5. The composition according to claim 4, wherein the radioisotope is Lu-177.

6. The composition according to claim 1, wherein the radio-immunoconjugate has an adsorption rate to the surface of vascular endothelial cells ranging from 5 to 30%.

7. The composition according to claim 6, wherein the adsorption rate ranges from 10 to 20%.

8. The composition according to claim 1, wherein the radio-immunoconjugate has an accumulation rate in cancer tissues of 2 to 10 times that in normal tissues.

9. The composition according to claim 8, wherein the accumulation rate in cancer tissues ranges from 3 to 5 times that in normal tissues.

10. The composition according to any one of claims 1 to 9, wherein the cancer is at least one selected from a group consisting liver cancer, gastric cancer, breast cancer, colon cancer, bone cancer, pancreatic cancer, head or neck cancer, uterine cancer, ovarian cancer, rectal cancer, esophageal cancer, small intestine cancer, perianal cancer, fallopian tube cancer, endometrial cancer, cervical cancer, vaginal cancer, vulvar cancer, Hodgkin's disease, prostate cancer, bladder cancer, renal cancer, ureter cancer, renal cytoma, renal pelvis cancer and tumors of central nervous system.

11. A method for detection of pre-metastatic sites, comprising: (1) administering the composition for detecting pre-metastatic sites as set forth in claim 1 to an individual with a cancer; and (2) detecting signals emitted from tissues of the individual by the composition of the step (1) then imaging the detected signals.

12. The method according to claim 11, wherein the imaging in the step (2) is performed by at least one selected from a positron emission tomography (PET), a photon emission computed tomography (SPECT) and a gamma camera.

13. A method for diagnosis of cancer or metastasis, comprising: (1) administering a composition containing radio-immunoconjugate having antibody marked with a radioisotope targeting a VEGFR to an individual; (2) detecting signals emitted from tissues of the individual by the composition in the step (1) then imaging the detected signals to determine an accumulation rate thereof; and (3) comparing the determined accumulation rate in the step (2) to that of a normal individual and selecting individuals with relatively high accumulation rates.

14. The method according to claim 13, wherein the accumulation rate in the step (2) ranges from 2 to 10 times that of a normal individual.

15. The method according to claim 14, wherein the accumulation rate ranges from 3 to 5 times that of a normal individual.

16. A kit for diagnosis of cancer or metastasis, containing a radio-immunoconjugate that has antibody marked with a radioisotope targeting a VEGFR.

17. A composition for inhibition of metastasis, containing a radio-immunoconjugate that has antibody marked with a radioisotope targeting a VEGFR.

18. A method for inhibition of metastasis, comprising administration of the composition for inhibition of metastasis as set forth in claim 17 in a pharmaceutically effective amount to an individual with a cancer.
